# EUROPEAN PATENT APPLICATION

(11) **EP 0 598 910 A1**
(43) Date of publication of application: **01.06.1994**
(21) Application number: 93905619.8
(22) Date of filing: 08.03.1993
(51) Int. Cl.: C07H 19/04, C07H 23/00, C07H 1/00, C07D 405/04

(54) **PROCESS FOR PRODUCING NUCLEOSIDE DERIVATIVE**

(30) Priority: 11.03.1992 JP 86545/92; 08.06.1992 JP 171550/92
(71) Applicant: Japan Tobacco Inc., Shinagawa-ku, Tokyo 140 (JP)
(72) Inventor: KAWAKAMI, Hiroshi, Japan Tobacco Inc., Yokohama-shi, Kanagawa-ken 227 (JP); EBATA, Takashi, Japan Tobacco Inc., Yokohama-shi, Kanagawa-ken 227 (JP); OKANO, Koji, Japan Tobacco Inc., Yokohama-shi, Kanagawa-ken 227 (JP); MATSUMOTO, Katsuya, Japan Tobacco Inc., Yokohama-shi, Kanagawa-ken 227 (JP); KOSEKI, Koshi, Japan Tobacco Inc., Yokohama-shi, Kanagawa-ken 227 (JP); MATSUSHITA, Hajime, Japan Tobacco Inc., Yokohama-shi, Kanagawa-ken 227 (JP)
(74) Representative: Ruffles, Graham Keith
(86) International application number: JP9300283
(87) International publication number: WO9318051

(57) **Abstract**

A process for producing a nucleoside derivative (1) by reacting a substituted 2,3-dihydrofuran derivative (2) with an organohalogen compound (3) containing a group 16 element of the periodic table to give a substituted 2- halotetrahydrofuran derivative (4), condensing the derivative (4) with a nucleic acid base to give a substituted nucleoside derivative (5), and reducing the derivative (5), wherein R¹ represents hydrogen, alkyl or R⁶⁻O-CH₂-; R² to R⁴ represent each hydrogen or a group containing a group 14-17 element of the periodic table; R⁵ represents optionally substituted alkyl or phenyl; A represents a group 16 element of the periodic table; B represents a nucleic acid base; and X represents halogen.

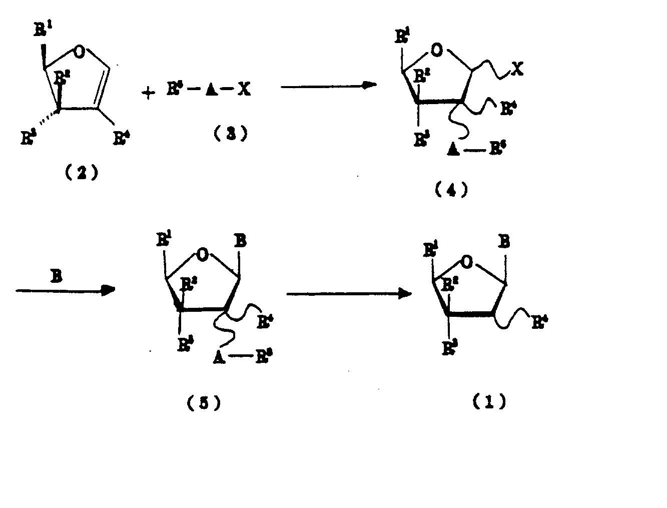

## Description

### [Title of the Invention)

### Process of producing a nucleoside derivative

### [Technical Field]

The present invention relates to a nucleoside derivative represented by a general formula (1), 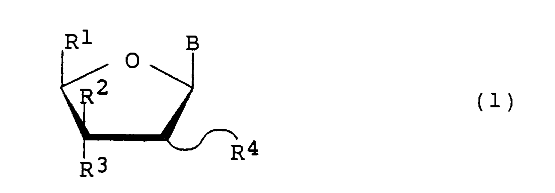 wherein, R¹, R², R³, R⁴, and B are defined as follows:
R¹ is a hydrogen atom, an alkyl group, or R⁶0CH₂-, with the proviso that R⁶ is a protecting group of a hydroxyl group,
R² is a hydrogen atom or a substituent made up of combinations of elements belonging to from the 14th group (the IV group) to the 17th group (the VII group) of the periodic table.
R³ is a hydrogen atom or a substituent made up of combinations of elements belonging to from the 14th group (the IV group) to the 17th group (the VII group) of the periodic table.
R⁴ is a hydrogen atom or a substituent made up of combinations of elements belonging to from the 14th group (the IV group) to the 17th group (the VII group) of the periodic table.
B is a nucleic acid base.

### [Prior Art)

A nucleoside derivative (1) is a nucleoside present in nature and a chemical compound having various physiological activities such as an anti-HIV activity, as well as a synthetic intermediate for manufacturing a pharmaceutically useful agent. Accordingly, the simple and efficient process of the present invention for synthesizing a compound (1) has significant advantages with regard to the developing of medicine.

Conventional methods of manufacturing a nucleoside derivative (1) include the following processes (1) to (3):
(1) A process using a conversion reaction of a functional group of ribonucleoside obtained from a natural source, i.e., 2'-deoxynucleoside;
(2) A process using a condensation reaction of a nucleic acid base with sugar; and
(3) A process using a conversion reaction of a functional group of a nucleoside intermediate obtained by a condensation reaction.

However, the process (1) is not suitable for a synthesis in a large scale since the amount of a nucleoside obtained from a natural source used as a raw material for this process is limited. In addition, the condition of the conversion reaction of a functional group is restricted due to the presence of a chemically labile glycoside-bond in the molecule. Using the process (2), the yield of a desired isomer is often low, since a stereoselectivity of the condensation reaction is different, depending on type of a sugar and nucleic acid base used. This process further has a drawback in that it requires an intricate operation process to isolate a desired isomer from the undesired isomers produced as by-product. The process (3) includes all the above-mentioned drawbacks since it is used in combination with the methods (1) and (2).

### [Disclosure of the Invention)

The present invention has been developed with a view toward providing a process of producing a nucleoside derivative (1) easily and efficiently.

According to one aspect of the present invention, there is provided a process of producing a compound represented by the following general formula (1) comprising a step (a), a step (b), and a step (c) described below.

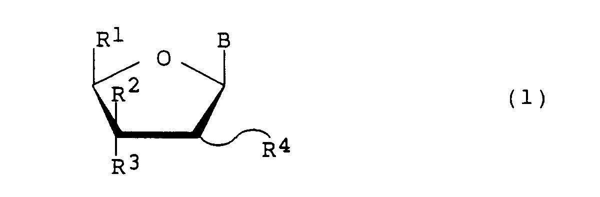

Wherein, R¹, R², R³, R⁴, and B are defined hereinabove. (a) is a step of producing a substituted 2-halogenotetrahydrofuran derivative represented by a general formula (4) by reacting a 2,3-dihydrofuran derivative represented by a general formula (2) with an organic halogenated compound represented by a general formula (3) containing an element (A) belonging to the 16th group (the VI group), as shown below.

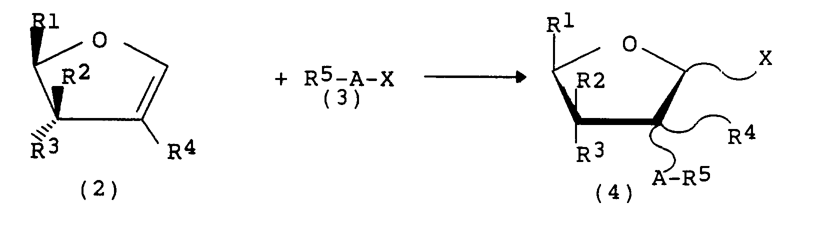

Wherein, the element (A) is an element belonging to the 16th group (the VI group) in the periodic table; X is a halogen atom; and R¹, R², R³, and R⁴ are defined hereinabove.

R⁵ is an alkyl group or a phenyl group, which may be substituted by functional group. (b) is a step of producing a substituted nucleoside derivative represented by a general formula (5) by condensing a compound (4) with a nucleic acid base (B), as shown below.

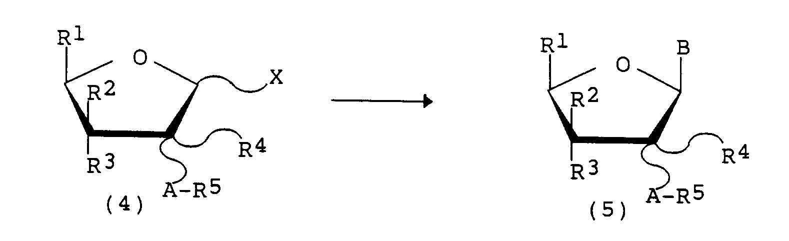

Wherein R¹, R², R³, R⁴, R⁵, A, B, and X are defined hereinabove. (c) is a step of producing a nucleoside derivative represented by a general formula (1) by reducing a compound (5), as shown below. 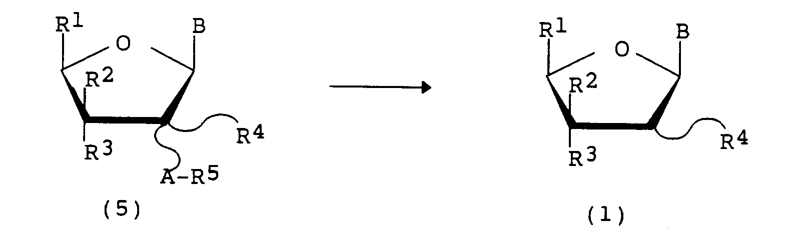 Wherein R¹, R², R³, R⁴, R⁵, A, and B are defined hereinabove.

Hereinafter, the present invention will be described in detail.

A starting material (2) used in the present invention can be industrially produced or prepared according to a method described in Tetrahedron, 1990, Vol. 46, Page 4503; J. Org. Chem., 1980, Vol. 45, Page 48; and J. Chem. Soc. Chem. Commun., 1986, Page 1149 and the like.

Wherein, a substituent R¹ of the substituted 2,3-dihydrofuran derivative (2) is a hydrogen atom, an alkyl group such as a methyl group and an ethyl group, or R⁶0CH₂-, wherein R⁶ is not restricted if it used for the purpose of protection of a hydroxyl group. Examples of R⁶ include an aralkyl group such as a benzyl group, and a trityl group; an acyl group such as an acetyl group, a propionyl group, a pivaloyl group, and a benzoyl group; an alkyloxycarbonyl group such as an ethoxy carbonyl group; an aryloxycarbonyl group such as a phenoxycarbonyl group; a triorganosilyl group such as a trimethylsilyl group, a t-butyldimethylsilyl group, and a t-butyldiphenylsilyl group. When these protecting groups contain a phenyl group, the phenyl group can be substituted by an functional group. The functional group on phenyl group is not restricted. Examples of the functional group include an alkyl group, a halogen atom, a nitro group, and an alkoxyl group.

In a reaction between a compound (2) and a compound (3), a substituent R^{S}A is bounded to the 4th position and a substituent X is bounded to the 5th position of the compound (2) by an electrophilic addition reaction. Substituents R² to R⁴ do not participate in this reaction. Therefore, substituents R² to R⁴ are acceptable if they do not interfere the electrophilic addition reaction. In other words, any substituent containing a hydrogen atom or an element belonging to from the 14th group (the IV group) to the 17th group (the VII group) of the periodic table can be employed as R², R³ and R^{4.} Examples of substituents include a substituent composed of a carbon atom, a substituent containing silicon and tin, a substituent containing nitrogen, a substituent containing phosphorus, a substituent containing oxygen, a substituent containing sulfur and selenium, and a substituent containing an halogen atom, a substituent containing a plurality of above-mentioned elements, and the like.

Representative examples will be mentioned herein-below, but should not be construed as limiting the scope of the present invention.

### (Substituent comprising carbon and the like)

An alkyl group such as a methyl group, an ethyl group, a propyl group, a butyl group, and cyclohexyl group; an aryl group such as a phenyl group and a naphthyl group; an aralkyl group such as a benzyl group and trityl group; an acyl group such as an acetyl group, benzoyl group; a carboxyl group; an amide group; a formyl group; and the above-mentioned groups substituted by a substituent containing a hetero atom such as a hydroxyalkyl group, a trialkylsilylmethyl group, a trialkylstannylmethyl group etc.; a cyano group, and the like.

### (Substituent containing silicon)

A trimethylsilyl group; t-butyldiphenylsilyl group; t-butyldimethylsilyl group; triphenylsilyl group, and the like.

### (Substituent containing tin)

A tributyltin, triphenyltin, and the like.

### (Substituent containing nitrogen)

An amino group such as a methylamino group, an ethylamino group, and a dimethylamino group; an azido group; a nitro group; a diazo group; and the like.

### (Substituent containing phosphorus)

A phosphono group; a phosphino group; and the like.

### (Substituent containing oxygen)

A hydroxy group, an alkyloxy group; an aryloxy group; a silyloxy group such as a trimethylsilyloxy group, a triethylsilyloxy group, t-butyldimethylsilyl oxy, t-butyldiphenylsilyloxy; an acyloxy; an aralkyloxy group such as benzyloxy; and the like.

### (Substituent containing sulfur)

A mercapto group; a sulphenyl group; a sulfinyl group; a sulfonyl group; and the like.

### (Substituent containing selenium)

An alkylseleno group such as a methylseleno group, an ethylseleno group; an arylseleno group such as a phenylseleno group; and the like.

### (Halogen atom)

Fluoride, chloride, bromide, and iodide.

R¹, R², R³, and R⁴ may be the same or different.

In the step (a), a substituted 2-halogenotetrahydrofuran derivative (4) is obtained from a substituted 2,3-dihydrofuran derivative (2) by an electrophilic addition reaction using a halogenated compound of an organic acid (3) containing a bivalent element belonging to the 16th group (the VI group) of the periodic table. As the method, known are methods described by Ibragimov et al., (Synthesis 1985, page 880), Alexander et al. (Tetrahedron Lett., 1983 Vol. 24, Page 5911) and the like. That is, a substituted 2,3-dihydrofuran derivative (2) is mixed with equimolar amount or above of said halogen compound (3) to the derivative (2) in an appropriate solvent with stirring thereby obtaining compound (4). A solvent to be used is not restricted if it is an aprotic solvent. Examples of the aprotic solvent include chloroform, methylene chloride, 1,2-dichloroethane, toluene, and the like. The reaction is usually completed under an anhydrous condition at room temperature or less for 0.5 to 5 hours.

In this step, the stereochemistry of R^{S}A to be introduced at the 4th position of the compound (2) is as shown below. Two types of isomers can be obtained; one is a trans-isomer (a compound 4a) and the other is a cis-isomer (a compound 4b) with respect to an R¹ group. In this reaction, the trans- and cis-isomer can be selectively obtained by appropriate selection of substituent R¹, R², R³, and R⁴. In order to obtain transisomer in a larger amount than cis-isomer, a compound having a larger group as R¹ may be employed, or a compound having a smaller group as R³ may be used. On the contrary, in order to obtain cis-isomer in a larger amount than trans-isomer, a compound having a more small group may be employed as R¹, or a compound having a more large group may be chosen as R³.

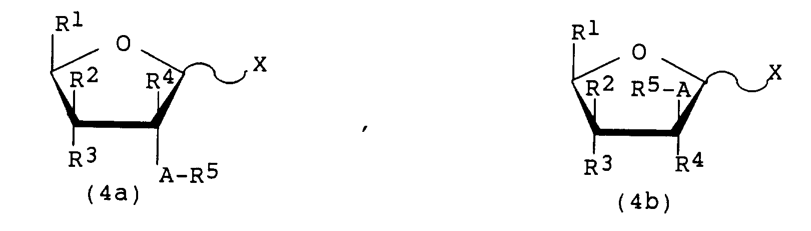

Wherein R¹, R², R³, R⁴, R⁵, A, and X are defined hereinabove.

Substituents R⁵ for a halogen compound (R^{S}AX) include an alkyl group such as a methyl group and a t-butyl group, an aralkyl group such as a benzyl group, an aryl group such as a phenyl group, and the like. When a substituent has a phenyl group, the phenyl group may have substituent. The substituent on phenyl group is not restricted. Examples of the substituent include an alkyl group, a halogen atom, a nitro group, an alkyloxy group, and the like. An element A is the 16th group (the VI group) in the periodic table such as oxygen, sulfur, selenium, and tellurium. In this invention, said four elements can be used, and sulfur and selenium can be preferably used. Examples of an element X include a fluoride, a chloride, a bromide, and an iodide atom, preferably a chloride and a bromide atom are used.

The resulting substituted 2-halogenotetrahydrofuran derivative (4) can be used in the next step (b) without an isolation and purification step.

In the step (b), a condensation reaction between the 2-halogenotetrahydrofuran derivative and a nucleic acid base is performed in an aprotic solvent by using one or more equivalent of the nucleic acid base to the 2-halogeneotatrahydrofuran derivative in the absence of a catalyst or in the presence of a Lewis acid. When the condensation reaction is performed in the absence of a catalyst, an obtained major isomer has stereochemistry of a cis-relationship between the substituent R^{S}A and the nucleic acid base B. On the other hand, when the condensation reaction is performed in the presence of a Lewis acid, an obtained major isomer has stereochemistry of a trans-relationship between said two groups. Accordingly, taking into account a configuration of the glycoside-bond of a desired nucleoside derivative (1) and configuration of a substituent R^{S}A of a substituted 2-halogenotetrahydrofuran derivative (4) obtained in the step (a), one of the above-mentioned two conditions is chosen in the condensation reaction. For example, in the case where it is desired to form a ,8-glycoside bond shown in a general formula (5), when using the compound (4a) obtained in a step (a) having an R^{S}A group in an a side thereof, the reaction may be performed in the presence of a Lewis acid (see the reaction [1] shown below). On the other hand, when using a compound (4b) having an R^{S}A group at a side, the reaction can be performed in the absence of a catalyst (see a reaction [2] shown below).

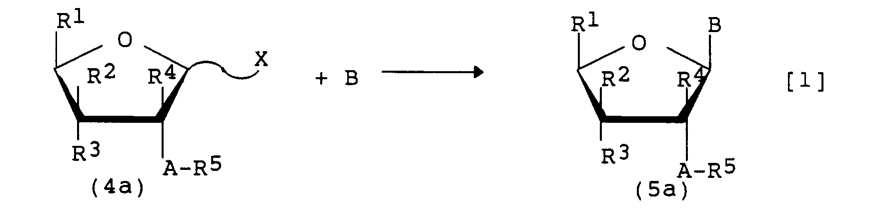

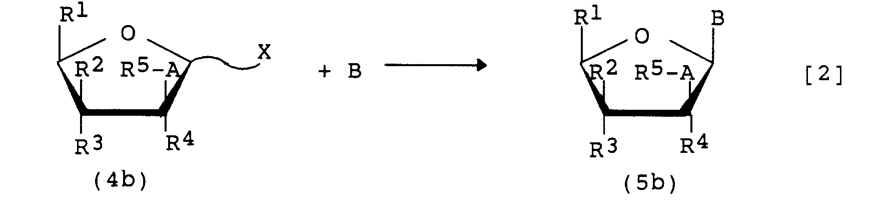

Wherein, R¹, R², R³, R⁴, R⁵, A, B, and X are defined hereinabove.

A Lewis acid to be used in the reaction is not restricted. A preferred Lewis acid to be used is trimethylsilyl trifluoromethansulfonate, tin tetrachloride, titanium tetrachloride, and the like. As a solvent to be used in the condensation reaction, aprotic solvent, e.g. chloroform, methylene chloride, 1,2-dichloroethane, acetonitrile, and the like is used. When a Lewis acid is employed as a catalyst, an effective amount of the Lewis acid in this reaction is 0.2 to 10 equivalent. This reaction is performed at room temperature or less and usually completed for 24 hours. After the completion of the reaction, the reaction mixture is treated with an alkaline solution such as a sodium hydrogencarbonate solution, if necessary, followed by extraction with an appropriate organic solvent and subjected to purification by means of a known method such as crystallization or chromatography, thereby obtaining a substituted nucleoside derivative (5).

In the step (b), a nucleic acid base can be used as it is or in an activated form thereof such as a silylated or a salt form. To make a silylated or a salt form of a nucleic acid base, the following methods can be employed, but should not be construed as limiting the scope of the present invention.

### (Method for making a silylated form of a nucleic acid base)

A silylation of a nucleic acid base can be performed according to a method described by Vorbruggen et al. (Chem. Ber. Vol. 114, 1234 (1981)). According to this method, trimethylsilylchloride is added to a nucleic acid base suspension in hexamethyldisilazane and the mixture is refluxed under an argon gas.

### (Method for making a salt form of a nucleic acid base)

There are the following two methods. One is a method disclosed in Published Unexamined Japanese Patent Application No. 2-17199 and Chem. Lett., 1989, Page 235) by the present inventors. This is a method in which 1 equivalent of a metal hydroxide or alcoholate is reacted with a nucleic acid base suspension in an alcohol or in a water.

The other is a method disclosed by Kazimierczuk et al. (J. Am. Chem. Soc., Vol. 106, 6379 (1984)). This is a method in which a salt is generated by reacting a metal hydride with a nucleic acid base in a reaction system.

The nucleic acid base include thymine, uracil, cytosine, adenine, guanine, hypoxthantine, xthantine which are obtained by decomposition from a nucleic acid present in nature, or a nucleic acid base synthesized from the above-mentioned nucleic acid base, and they can be used in this invention. Further, a completely synthesized nucleic acid base which is not derived from a natural source can be also used.

A reduction reaction of a substituted nucleoside (5) in a step (c) is performed in the presence of a metal catalyst or an organic metal hydride.

A metal catalyst is not restricted if it can remove an R^{S}A group reductively. For example, a catalyst containing nickel, preferably Raney nickel and the like can be used. The reduction reaction is performed at a range of room temperature to 100°C, preferably from room temperature to 85 ° C. Although a solvent to be used in the reaction is not particularly restricted, preferably alcohol, more preferably ethanol can be used.

Although an organic metal hydride is not restricted if it can remove an R^{S}A group reductively. Tributyltin hydride, triphenyltin hydride, diphenylsilane, and the like can be used. In this reduction reaction by use of an organic metal hydride compound, the reaction is effectively proceeded if a compound known as a radical initiator is added as a catalyst. As examples of the radical initiator, there may be mentioned benzoyl peroxide, azobisisobutyronitrile (AIBN), triethylborane, and the like. Although a suitable amount of the radical initiator is different in an individual reaction, preferably 5 to 20 % by mol of the substituted nucleoside. The reaction is performed using an organic metal hydride in an equivalent amount or more, preferably from 2 to 5 equivalent, relative to a compound (5) at room temperature to 150°C for 1 to 24 hours. Although a solvent is not restricted if it is not a radical scavenger, preferably a hydrocarbon solvent such as benzene and toluene is used.

Further, the reduction can be performed under a condition in which an organic metal hydride can be generated in a reaction solution. Example is a reaction performed in the co-presence of bis(tributyltin)oxide and polymethylhydrosiloxane.

The reaction mixture obtained by the above reduction reaction is purified by means of a known method such as chromatography to give a desired nucleoside derivative (1) in pure form.

A nucleoside derivative in the present invention may be compound having various physiological activities of a nucleoside present in nature, anti-HIV activity and the like, and can be a synthetic intermediate for manufacturing a useful compound in a medicine. According to the present invention, the nucleoside derivative can be selectively obtained in a convenient process, and therefore can be constantly provided in a large amount.

### [The Best Embodiments to Carried Out the Invention)

Hereinafter, the present invention will be described in more detail with reference to the following Examples.

### Example 1

### Synthesis of 1-(3-phenylthiotetrahydrofuran-2-yl)uracil

1 ml of anhydrous methylene chloride solution containing 41 µl (0.54 mmol) of 2,3-dihydrofuran was added dropwise to 3 ml of anhydrous methylene chloride solution containing 83 mg (0.58 mmol) of benzenesulfenyl chloride at -78 _{°} C under an argon atmosphere. Then the mixed solution was stirred for 30 minutes at room temperature, followed by a cooling down to -78 °C. Then, 2.2 ml (2.2 mmol) of methylene chloride solution containing 1.0 mol/I tin tetrachloride was added dropwise thereto. Further, 2.0 ml (2.0 mmol) of methylene chloride solution containing 1 mol/I 2,4-bis(trimethylsiloxy)pyrimidine was added thereto. The temperature was gradually raised to 0 _{°} C and the mixture was subsequently stirred for 2 hours. After the completion of the reaction, the reaction solution was poured into a saturated aqueous solution of sodium hydrogencarbonate and extracted with chloroform. The organic layer obtained from the chloroform extraction was dried over anhydrous magnesium sulfate and removed a solvent by evaporation under reduced pressure, thereby obtaining a residue.

The obtained residue was purified by means of preparative silica gel chromatography (an eluting solution is a mixed solution of chloroform and aceton in a ratio of 85 : 15). The purified product contains desired 1-(3-trans-phenylthiotetrahydrofuran-2-yl)uracil and its stereoisomer in a ratio of 99 : 1, and a yield thereof was 125 mg (80 %).

Physical properties of a trans-isomer in the obtained product are as follows:
^{I}H-NMR (CDCI₃): 6 (ppm)
9.54 (1 H, br, NH)
7.56 - 7.48 (2H, m, aromatic H)
7.36 - 7.26 (3H, m, aromatic H)
7.15 (1 H, d, J = 8.1 Hz, H - 6)
5.82 (1 H, d, J = 3.8 Hz, H - 2')
5.65 (1 H, d, J = 8.1 Hz, H - 5)
4.32 - 4.10 (1 H, m, H - 5')
4.17 (1H, q, J = 7.8 Hz, H - 5')
3.94 (1 H, ddd, J = 7.0, 5.3, 3.7 Hz, H - 3')
2.38 (1 H, dt, J = 20.7, 7.4 Hz, H - 4')
2.16 - 2.02 (1 H, m, H - 4')

### Example 2

### Synthesis of 1-(3-phenylthiotetrahydrofuran-2-yl)uracil

1 ml of anhydrous methylene chloride solution containing 50 µl (0.66 mmol) of 2,3-dihydrofuran was added dropwise to 3 ml of anhydrous methylene chloride solution containing 101 mg (0.70 mmol) of benzenesulfenyl chloride at -50 ° C under an argon atmosphere. Then, the mixture was stirred for 30 minutes at -50 ° C, followed by cooling down to -78 ° C. Then, 2.0 ml (2.0 mmol) of methylene chloride solution containing 1.0 mol/I 2,4-bis(trimethylsiloxy)pyrimidine was added thereto. The temperature was gradually raised to room temperature and the mixture was subsequently stirred for 22 hours. After the completion of the reaction, the reaction solution was poured into a saturated aqueous solution of sodium hydrogencarbonate and extracted with chloroform. The organic layer obtained from the chloroform extraction was dried over anhydrous magnesium sulfate and removed a solvent by evaporation under reduced pressure, thereby obtaining a residue.

The obtained residue was purified by means of preparative silica gel chromatography (an eluant is a mixed solution of chloroform and aceton in a ratio of 80 : 20). The purified product contains desired 1-(3- cis-phenylthiotetrahydrofuran-2-yl)uracil and stereoisomer thereof in a ratio of 85 : 15. A yield of the purified product was 159 mg (82 %).

Physical properties of a cis-isomer in the obtained product are as follows:
¹H-NMR (CDCI₃): 6 (ppm)
9.06 (1 H, br, NH)
7.53 (1 H, d, J = 8.0Hz, H - 6)
7.36 - 7.15 (5H, m, aromatic H)
6.18 (1 H, d, J = 4.9 Hz, H - 2')
5.70 (1 H, d, J = 8.1 Hz, H - 5)
4.35 (1 H, dt, J = 7.5 Hz, 4.5 Hz, H - 5')
4.34 - 4.35 (1 H, m, H - 5')
4.01 (1 H, dt, J = 8.2, 5.0 Hz, H - 3')
2.60 (1 H, dq, J = 13.6, 7.6 Hz, H - 4')
2.24 - 2.12 (1H, m, H - 4')

### Example 3

### Synthesis of 1-(3-phenylselenotetrahydrofuran-2-yl)uracil

1 ml of anhydrous methylene chloride solution containing 55 µl (0.73 mmol) of 2,3-dihydrofuran was added dropwise to 3 ml of anhydrous methylene chloride solution containing 141 mg (0.74 mmol) of benzeneselenenyl chloride at -50 ° C under an argon atmosphere. Then, the mixture was stirred for 30 minutes at -50 ° C, followed by cooling down to -78 ° C. Then, 1.6 ml (1.6 mmol) of methylene chloride solution containing 1.0 mol/I tin tetrachloride was added thereto. Then, 1.4 ml (1.4 mmol) of methylene chloride solution containing 1.0 mol/I 2,4-bis(trimethylsiloxy)pyrimidine was added thereto. The temperature was gradually raised to 0 ° C and the mixture was subsequently stirred for 2 hours. After the completion of the reaction, the reaction solution was poured into a saturated aqueous solution of sodium hydrogencarbonate and extracted with chloroform. The organic layer obtained from the chloroform extraction was dried over anhydrous magnesium sulfate and removed a solvent by evaporation under reduced pressure, thereby obtaining a residue.

The obtained residue was purified by means of preparative silica gel chromatography (an eluant is a mixed solution of chloroform and aceton in a ratio of 80 : 20). The purified product contains desired 1-(3- trans-phenylselenotetrahydrofuran-2-yl)uracil and stereoisomer thereof in a ratio of 98 : 2. A yield of the purified product was 94 mg (38 %).

Physical properties of a trans-isomer in the obtained product are as follows:
¹H-NMR (CDCI₃): 6 (ppm)
8.97 (1 H, br, NH)
7.69 - 7.63 (2H, m, aromatic H)
7.36 - 7.26 (3H, m, aromatic H)
7.15 (1 H, d, J = 8.1 Hz, H - 6)
5.94 (1 H, d, J = 4.3 Hz, H - 2')
5.65 (1 H, dd, J = 8.1, 1.7 Hz, H - 5)
4.28 - 4.21 (1 H, m, H - 5')
4.12 (1 H, q, J = 7.6 Hz, H - 5')
3.94 (1 H, ddd, J = 7.0, 5.9, 4.4 Hz, H - 3')
2.40 (1 H, dt, J = 20.7, 7.2 Hz, H - 4')
2.19 - 2.08 (1 H, m, H - 4')

### Example 4

### Synthesis of N⁶⁻benzoyl-9-(3-transphenylthiotetrahydrofuran-2-yl)adenine

1 ml of anhydrous methylene chloride solution containing 38 µl (0.50 mmol) of 2,3-dihydrofuran was added dropwise to 2.5 ml (0.50 mmol) of methylene chloride solution containing 0.20 mol/I benzenesulfenyl chloride at -78 ° C under an argon atmosphere. Then, the mixture was stirred for 30 minutes at -78 ° C. Then, 1.3 ml (1.3 mmol) of methylene chloride solution containing 1.0 mol/I tin tetrachloride was added dropwise thereto. Further, 2 ml of anhydrous methylene chloride solution containing 1.0 mmol of 6-(N-benzoyl-N-trimethylsilyl)amino-9-trimethylsilylpurine was added thereto. The temperature was gradually raised to 0 ° C and the mixture was subsequently stirred for 2 hours. After the completion of the reaction, the reaction solution was poured into a saturated aqueous solution of sodium hydrogencarbonate and extracted with chloroform. The organic layer obtained from the chloroform extraction was dried over anhydrous magnesium sulfate and removed a solvent by evaporation under reduced pressure, thereby obtaining a residue.

The obtained residue was purified by means of preparative silica gel chromatography (an eluant is a mixed solution of chloroform and aceton in a ratio of 85 : 15). Desired N⁶⁻benzoyl-9-(3-trans-phenyl- thiotetrahydrofuran-2-yl)adenine was obtained in a yield of 107 mg (51 %).

Physical properties of the obtained product are as follows:
¹H-NMR (CDCI₃): 6 (ppm)
9.05 (1 H, br, NH)
8.78 (1 H, s, H - 8)
8.08 - 8.00 (2H, m, aromatic H)
7.90 (1 H, s, H - 2)
7.65 - 7.49 (3H, m, aromatic H)
7.45 - 7.36 (2H, m, aromatic H)
7.30 - 7.20 (3H, m, aromatic H)
6.08 (1 H, d, J = 3.9 Hz, H - 2')
4.64 (1 H, ddd, J = 7.4, 6.0, 3.9 Hz, H - 3')
4.44 (1 H, ddd, J = 8.4, 7.4, 6.4 Hz, H - 5')
4.32 - 4.23 (1 H, m, H - 5')
2.71 (1 H, dt, J = 13.5, 7.3 Hz, H - 4')
2.23 - 2.12 (1 H, m, H - 4')

### Example 5

### Synthesis of 1-[5-O-(t-butyldiphenylsilyl)-2,3-dideoxy-2-phenylthio-β-D-erythro-pentofuranosyl]uracil

1.4 ml of anhydrous methylene chloride solution containing 48 mg (0.14 mmol) of 2-(t-butyldiphenyl- silyloxymethyl)-2,3-dihydrofuran was added dropwise to 1.4 ml (0.15 mmol) of methylene chloride solution containing 0.11 mol/I benzenesulfenyl chloride at -78 °C under an argon atmosphere. Then, the mixture was stirred for 30 minutes at -78 ° C. Then, 0.37ml (0.37 mmol) of methylene chloride solution containing 1.0 mol/I tin tetrachloride was added dropwise thereto at -78 ° C. After addition was completed, 1.4 ml (0.29 mmol) of anhydrous methylene chloride solution containing 0.20 mol/I 2,4-bis(trimethylsiloxy)pyrimidine was added thereto. The temperature was gradually raised to 0 ° C and the mixture was subsequently stirred for 1 hour. After the completion of the reaction, the reaction solution was poured into a saturated aqueous solution of sodium hydrogencarbonate and extracted with chloroform. The organic layer obtained from the chloroform extraction was dried over anhydrous magnesium sulfate and removed a solvent by evaporation under reduced pressure, thereby obtaining a residue.

The obtained residue was purified by means of preparative silica gel chromatography (an eluant is a mixed solution of n-hexane and ethyl acetate in a ratio of 50 : 50). Desired 1-[5-0-(t-butyldiphenylsilyl)-2,3- dideoxy-2-phenylthio-β-D-erythro-pentofuranosyl]uracil was obtained in a yield of 62 mg (77 %).

Physical properties of the obtained product are as follows:
¹H-NMR (CDCI₃): 6 (ppm)
8.97 (1 H, br, NH)
7.72 (1 H, d, J = 8.1, H - 6)
7.67 - 7.61 (4H, m, aromatic H)
7.50 - 7.36 (8H, m, aromatic H)
7.28 - 7.24 (3H, m, aromatic H)
6.08 (1 H, d, J = 5.5 Hz, H - 1')
5.31 (1 H, dd, J = 8.1, 2.0 Hz, H - 5)
4.38 - 4.30 (1 H, m, H - 4')
4.08 (1 H, dd, J = 11.7, 2.1 Hz, H - 5')
3.90 - 3.73 (1 H, m, H - 2')
3.69 (1 H, dd, J = 11.7, 2.2 Hz, H - 5')
2.52 (1 H, ddd, J = 13.2, 7.2, 6.0 Hz, H - 3')
2.10 (1 H, dt, J = 13.1, 7.4 Hz, H - 3')
1.10 (9H, s, t-Bu)

### Example 6

### Synthesis of 1-[5-O-(t-butyldiphenylsilyl)-2,3-dideoxy-2-phenylthio-β-D-erythropentofuranosyl]thymine

10.5 ml of anhydrous methylene chloride solution containing 350 mg (1.05 mmol) of 2-(t-butyldiphenyl- silyloxymethyl)-2,3-dihydrofuran was added dropwise to 10.5 ml (1.08 mmol) of methylene chloride solution containing 0.103 mol/I benzenesulfenyl chloride at 0 ° C under an argon atmosphere. Then, the mixture was stirred for 30 minutes at 0 ° C, followed by cooling down to -78 °C. 2.3 ml (2.3 mmol) of methylene chloride solution containing 1.0 mol/I tin tetrachloride was added dropwise thereto. Further, 10.5 ml (2.1 mmol) of methylene chloride solution containing 0.20 mol/I 5-methyl-2,4-bis(trimethylsiloxy)pyrimidine was added thereto. The temperature was gradually raised to 0 ° C and the mixture was subsequently stirred for 2 hours. After the completion of the reaction, the reaction solution was poured into a saturated aqueous solution of sodium hydrogencarbonate and extracted with chloroform. The organic layer obtained from the chloroform extraction was dried over anhydrous magnesium sulfate and removed a solvent by evaporation under reduced pressure, thereby obtaining a residue.

The obtained residue was purified by means of preparative silica gel chromatography (an eluant; n-hexane : ethyl acetate = 75 : 25 to 50 : 50). The purified product was recrystallized from the mixed solvent of n-hexane and methylene chloride, thereby obtaining desired 1-[5-O-(t-butyldiphenylsilyl)-2,3-dideoxy-2-phenylthio-β-D-erythro-pentofuranosyl]thymine in a yield of 318 mg (0.555 mmol) (53 %).

Physical properties of the obtained product are as follows:
¹H-NMR (CDCI₃): 6 (ppm)
8.96 (1 H, br, NH)
7.70 - 7.62 (4H, m, aromatic H)
7.52 - 7.33 (8H, m, aromatic H)
7.27 - 7.15 (4H, m, H - 6, aromatic H)
6.12 (1 H, d, J = 7.6 Hz, H - 1')
4.28 - 4.21 (1 H, m, H - 4')
4.02 (1 H, dd, J = 11.5, 1.8 Hz, H - 5')
3.89 - 3.72 (1 H, m, H - 2')
3.68 (1 H, dd, J = 11.5, 2.1 Hz, H - 5')
2.54 (1 H, ddd, J = 12.5, 8.0, 4.2 Hz, H - 3')
2.14 (1 H, dt, J = 12.9, 9.2 Hz, H - 3')
1.50 (3H, s, Me)
1.13 (9H, s, t-Bu)

### Example 7

### Synthesis of N⁴-acetyl-1-[5-O-(t-butyldiphenylsilyl)-2,3-dideoxy-2-phenylthio-β-D-erythro-pentofuranosyl]-cytosine

10 ml of anhydrous methylene chloride solution containing 341 mg(1.03 mmol) of 2-(t-butyldiphenyl- silyloxymethyl)-2,3-dihydrofuran was added dropwise to 10.0 ml (1.00 mmol) of methylene chloride solution containing 0.100 mol/I benzenesulfenyl chloride at 0 ° C under an argon atmosphere. Then, the mixture was stirred for 30 minutes at 0 ° C, followed by cooling down to -78 ° C. 2.2 ml (2.2 mmol) of methylene chloride solution containing 1.0 mol/I tin tetrachloride was added dropwise thereto. Further, 10 ml (2.0 mmol) of methylene chloride solution containing 0.2 mol/I 4-(N-trimethylsilylacetamido)-2-trimethylsiloxypyrimidine was added thereto. The temperature was gradually raised to 0 ° C and the mixture was subsequently stirred for 2 hours. After the completion of the reaction, the reaction solution was poured into a saturated aqeous solution of sodium hydrogencarbonate and extracted with chloroform. The organic layer obtained from the chloroform extraction was dried over anhydrous magnesium sulfate and removed a solvent by evaporation under reduced pressure, thereby obtaining a residue.

The obtained residue was purified by means of preparative silica gel chromatography (an eluant is a mixed solution of chloroform and acetone in a ratio of 80 : 20). The purified product was further purified by means of high performance liquid chromatography (a fixed phase: A-363, ODS, has a diameter of 30 mm and a length of 250 mm, manufactured and sold by YMC Co., Ltd.; a moving phase consists of acetonitrile and water in a ratio of 75 : 25, a flow rate: 15 ml/minutes; a detective wavelength of ultraviolet rays: X = 254 nm), thereby obtaining desired N⁴-acetyl-1-[5-O-(t-butyldiphenylsilyl)-2,3-dideoxy-2-phenylthio-β-D-erythro-pentofuranosyl]cytosine in a yield of 441 mg (0.743 mmol) (72 %).

Physical properties of the obtained product are as follows:
¹H-NMR (CDCI₃): 6 (ppm)
10.18 (1 H, br, NH)
8.34 (1 H, d, J = 7.5 Hz, H - 6)
7.68 - 7.61 (4H, m, aromatic H)
7.50 - 7.35 (8H, m, aromatic H)
7.30 - 7.24 (3H, m, aromatic H)
7.20 (1 H, d, J = 7.5 Hz, H - 5)
6.08 (1 H, d, J = 2.6 Hz, H - 1')
4.55 - 4.45 (1 H, m, H - 4')
4.18 (1 H, dd, J = 11.9, 2.2 Hz, H - 5')
3.88 (1 H, dt, J = 6.3, 3.0 Hz, H - 2')
3.72 (1 H, dd, J = 12.0, 2.6 Hz, H - 5')
2.40 (1 H, ddd, J = 13.3, 9.2, 6.6 Hz, H - 3')
2.27 (3H, s, Ac)
1.94 (1 H, ddd, J = 13.3, 5.7, 3.2 Hz, H - 3')
1.11 (9H, s, t-Bu)

### Example 8

### Synthesis of N⁶-benzoyl-9-(5-O-pivaloyl-2,3-dideoxy-2-phenylthio-β-D-erythro-pentofuranosyl)adenine

1.6 ml of anhydrous methylene chloride solution containing 29 mg(0.16 mmol) of 2-(pivaloyloxymethyl)-2,3-dihydrofuran was added dropwise to 1.6 ml (0.16 mmol) of methylene chloride solution containing 0.10 mol/I benzenesulfenyl chloride at -78 ° C under an argon atmosphere. Then, the mixture was stirred for 30 minutes at -78 ° C. 0.4 ml (0.4 mmol) of methylene chloride solution containing 1.0 mol/I tin tetrachloride was added dropwise thereto. Further 1.6 ml (0.32 mmol) of a methylene chloride solution containing 0.20 mol/I 6-(N-benzoyl-N-trimethylsilyl)amino-9-trimnethylsilylpurine was added. Then, temperature was gradually raised to 0 ° C and the mixture was stirred for 5 hours. After the completion of the reaction, the reaction solution was poured into a saturated aqueous solution of sodium hydrogencarbonate and extracted with chloroform. The organic layer obtained from the chloroform extraction was dried over anhydrous magnesium sulfate and removed a solvent by evaporation under reduced pressure, thereby obtaining a residue.

The obtained residue was purified by means of preparative silica gel chromatography (an eluant is a mixed solution of chloroform and acetone in a ratio of 90 : 10). Desired N⁶⁻benzoyl-9-(5-O-pivaloyl-2,3- dideoxy-2-phenylthio-βD-erythro-pentofuranosyl)adenine was obtained in a yield of 59 mg (71 %).

Physical properties of the obtained product are as follows:
¹H-NMR (CDCI₃): 6 (ppm)
9.14 (1 H, br, NH)
8.76 (1 H, s, H - 8)
8.07 - 8.00 (3H, m, 2-H, aromatic H)
7.65 - 7.58 (1 H, m, aromatic H)
7.58 - 7.48 (2H, m, aromatic H)
7.42 - 7.37 (2H, m, aromatic H)
7.25 - 7.18 (3H, m, aromatic H)
6.09 (1 H, d, J = 4.2 Hz, H - 1')
4.69 - 4.57 (2H, m, H - 2', H - 4')
4.34 (2H, d, J = 4.6 Hz, H - 5')
2.63 (1 H, dt, J = 13.5, 7.7 Hz, H - 3')
2.24 (1 H, ddd, J = 13.4, 6.8, 5.1 Hz, H - 3')
1.21 (9H, s, t-Bu)

### Example 9

### Synthesis of N²-acetyl-9-(5-O-pivaloyl-2,3-dideoxy-2-phenylthio-β-D-erythro-pentofuranosyl)guanine

1.1 ml of anhydrous methylene chloride solution containing 21 mg (0.11 mmol) of 2-(pivaloyloxymethyl)-2,3-dihydrofuran was added dropwise to 1.1 ml (0.11 mmol) of methylene chloride solution containing 0.10 mol/I benzenesulfenyl chloride at -78 ° C under an argon atmosphere. Then, the mixture was stirred for 30 minutes at -78 ° C. 0.25 ml (0.25 mmol) of methylene chloride solution containing 1.0 mol/I tin tetrachloride was added dropwise thereto at -78 °C. Further, 1.1 ml (0.23 mmol) of methylene chloride solution containing 0.21 mol/I 2-(N-acetyl-N-trimethylsilyl)amino-9-trimethylsilyl-6-trimethylsiloxypurine was added to the above mixture. Then, temperature was gradually raised to 0 ° C and the mixture was subsequently stirred for 5 hours. After the completion of the reaction, the reaction solution was poured into a saturated aqueous solution of sodium hydrogencarbonate solution and extracted with chloroform. The organic layer obtained from the chloroform extraction was dried over anhydrous magnesium sulfate and removed a solvent by evaporation under reduced pressure, thereby obtaining a residue.

The obtained residue was purified by means of preparative silica gel chromatography (an eluant: a mixed solution of chloroform and acetone in a ratio of from 70 : 30). Desired N²⁻acetyl-9-(5-O-pivaloyl-2,3- dideoxy-2-phenylthio-β-D-erythropentofuranosyl)guanine was obtained in a yield of 13 mg (24 %).

Physical properties of the obtained product are as follows: 'H-NMR (CDCI₃): 6 (ppm)
11.92 (1 H, br, NH)
9.54 (1 H, br, NH)
7.45 (1 H, s, H - 8)
7.36 - 7.23 (5H, m, aromatic H)
5.83 (1 H, d, J = 4.3 Hz, H - 1')
4.88 (1 H, dd, J = 11.1, 6.4 Hz, H - 5')
4.68 (1 H, quint, J = 6.5 Hz, H - 4')
4.33 (1 H, dd, J = 11.1, 6.3 Hz, H - 5')
4.20 (1 H, ddd, J = 7.5, 5.9, 4.3 Hz, H - 2')
2.70 - 2.61 (1 H, m, H - 3')
2.33 (1 H, s, Ac)
2.24 (1 H, ddd, J = 13.2, 7.0, 6.1 Hz, H - 3')
1.22 (9H, s, t-Bu)

### Example 10

### Synthesis of 1-[3,5-O-bis(triethylsilyl)-2-deoxy-2-phenylthio-D-xylo-pentofuranosyl]uracil

1.8 ml of anhydrous methylene chloride solution containing 32 mg (0.091 mmol) of 3-(t-butyldimethyl- silyloxy)-2-(t-butyldimethysilyoxymethyl)-2,3-dihydrofuran was added dropwise to 0.9 ml (0.09 mmol) of methylene chloride solution containing 0.10 mol/I benzenesulfenyl chloride at -78 °C under an argon atmosphere. Then, the mixture was stirred for 30 minutes at -78°C. 0.25 ml (0.25 mmol) of methylene chloride solution containing 1.0 mol/I tin tetrachloride was added dropwise thereto at -78 ° C. Thereafter, further 0.9 ml (0.18 mmol) of methylene chloride solution containing 0.20 mol/I 2,4-bis(trimethylsiloxy)-pyridine was added to the above mixture. Then, the mixture was refluxed for 1.5 hours. After the completion of the reaction, the reaction solution was poured into a saturated aqueous solution of sodium hydrogencarbonate and extracted with chloroform. The organic layer obtained from the chloroform extraction was dried over anhydrous magnesium sulfate and removed a solvent by evaporation under reduced pressure, thereby obtaining a residue.

The obtained residue was dissolved in 3 ml of tetrahydrofuran, and then 0.25 ml of tetrahydrofuran solution containing 1.0 mmol/I tetrabutylammonium fluoride was added thereto and stirred for 40 minutes at room temperature. After the completion of the reaction, a cation exchange resin (Amberlite IR-120B pyridinium-form) was added to the mixture to neutralize the mixture. After the resin was filtered off, a solvent was removed reduced pressure. The residue was dissolved in 4 ml of anhydrous N,N-dimethylformamide. To this solution, 70 mg (1.0 mmol) of imidazole and 0.16 ml (0.95 mmol) of triethylsilylchloride were added in that order under dry condition with anhydrous calcium chloride. After stirring at room temperature for 2 days, the reaction mixture was poured into an aqueous solution of sodium chloride and extracted with chloroform. The organic layer obtained from the chloroform extraction was dried over anhydrous magnesium sulfate and a solvent was removed under reduced pressure.

The residue was purified by means of preparative silica gel chromatography (an eluant is a mixed solution of n-hexane and ethyl acetate in a ratio of from 60 : 40). Obtained 1-[3,5-O-bis-(triethylsilyl)-2-deoxy-2-phenylthio-D-xylo-pentofuranosyl]uracil in a yield of 31 mg (60 %) was the mixture consisting of an a - and a β-isomers in a ratio of 1 to 3.

Physical properties of the obtained product are as follows:
¹H-NMR(CDCI₃): δ (ppm)
8.46 (0.75H, br, NH)
8.21 (0.25H, br, NH)
7.76 (0.75H, d, J = 8.2 Hz, H - 6)
7.60 (0.25H, d, J = 8.2 Hz, H - 6)
7.58 - 7.51 (1.5H, m, aromatic H)
7.39 - 7.25 (3.5H, m, aromatic H)
6.36 (0.25H, d, J = 4.7 Hz, H - 1')
6.05 (0.75H, d, J = 2.6 Hz, H - 1')
5.70 (0.25H, d, J = 8.2 Hz, H - 5)
5.63 (0.75H, d, J = 8.2 Hz, H - 5)
4.49 - 4.30 (1.25H, m, H - 2', H - 4')
4.20 (0.75H, dd, J = 3.5, 2.0 Hz, H - 3')
4.12 (0.25H, dd, J = 4.7, 1.5 Hz, H - 3')
3.99 (1.5H, d, J = 5.6 Hz, H - 5')
3.84 (0.5H, d, J = 5.6 Hz, H - 5')
3.54 (0.75H, t, J = 2.3 Hz, H - 2')
1.05 - 0.89 (12H, m, methyl)
0.89 - 0.79 (6H, m, methyl)
0.71 - 0.50 (8H, m, methylene)
0.50 - 0.39 (4H, m, methylene)

### Example 11

### Synthesis of 1-[5-O-(t-butyldiphenylsilyl)-2,3-dideoxy-D-glycero-pentofuranosyl]uracil

300 mg of Raney nickel (W-2) was added to 10 ml of ethanol containing 230 mg (0.41 mmol) of 1-[5-0-(t-butyldiphenylsilyl)-2,3-dideoxy-2-phenylthio-D-erythropentofuranosyl]uracil (α : β = 21 : 79). The mixture was refluxed for 9 hours. After the completion of the reaction, an insoluble material was filtered off by using Celite. A solvent was removed by evaporation under reduced pressure, thereby obtaining a residue.

The obtained residue was purified by means of preparative silica gel chromatography (an eluant is a mixed solution of chloroform and methanol in a ratio of 96 : 4). A desired compound was obtained in a yield of 59 mg (32 %) in the form of a mixture containing an a-and a β-isomer in a ratio of 23 : 77.

Physical properties of the obtained β-isomer are as follows:
¹H-NMR (CDCl₃): 6 (ppm)
8.87 (1 H, br, NH)
7.99 (1 H, d, J = 8.1 Hz, H - 6)
7.70 - 7.62 (4H, m, aromatic H)
7.48 - 7.36 (6H, m, aromatic H)
6.11 (1H, dd, J = 6.6, 2.8 Hz, H - 1')
5.41 (1 H, dd, J = 8.1, 2.1 Hz, H - 5)
4.17 - 4.09 (2H, m, H - 4', H - 5')
3.73 (1 H, dd, J = 11.5, 2.6 Hz, H - 5')
2.51 - 2.35 (1 H, m, H - 2')
2.18 - 2.04 (2H, m, H - 2', H - 3')
1.98 - 1.88 (1 H, m, H - 3')
1.09 (9H, s, t-Bu)

### Example 12

### Synthesis of 9-(5-O-pivaloyl-2,3-dideoxy-β-D-glycero-pentofuranosyl)adenine

0.10 ml of polymehtylhydrosiloxane and 0.13 ml (0.25 mmol) of bis(tributyltin)oxide were added to 9 ml of toluene solution containing 24 mg (0.045 mmol) of N⁶⁻benzoyl-9-(5-O-pivaloyl-2,3-dideoxy-2-phenylthio-β-D-erythro-pentofuranosyl]adenine. The mixture was refluxed for 17 hours under an argon atmosphere while adding 50 µl (1.2 µmol) portions of toluene solution containing 23 mmol/I azobisisobutyronitrile every 30 minutes. After the completion of the reaction, a solvent was removed under reduced pressure, thereby obtaining a residue.

The obtained residue was purified by means of silica-gel column chromatography (an eluant; chloroform : methanol = 100 : 0 to 93 : 7), thereby removing polymer and tin compounds. Fractions containing the desired product were collected and purified by means of preparative silica gel chromatography (an eluant is a mixed solution of chloroform and methanol in a ratio of 95 : 5). A desired compound was obtained in a yield of 10 mg (72 %).

Physical properties of the obtained product are as follows:
¹H-NMR (CDCl₃): 6 (ppm)
8.35 (1 H, s, H - 8)
8.07 (1 H, s, H - 2)
6.29 (1 H, dd, J = 5.9, 4.0 Hz, H - 1')
5.78 (2H, br, NH)
4.47 - 4.37 (1 H, m, H - 4')
4.32 (2H, d, J = 4.4 Hz, H - 5')
2.67 - 2.50 (2H, m, H - 2')
2.23 - 1.98 (2H, m, H - 3')
1.22 (9H, s, t-Bu)

### Example 13

### Synthesis of 1-[3,5-O-bis(triethylsilyl)-2-deoxy-β-D-threo-pentofuranosyl]uracil

0.20 ml of polymehtylhydrosiloxane and 0.25 ml (0.25 mmol) of bis(tributyltin)oxide were added to 10 ml of toluene solution containing 49 mg (0.087 mmol) of 1-[3,5-O-bis(triethylsilyl)-2-deoxy-2-phenylthio-D-xylo-pentofuranosyl]uracil a- : β-isomer = 1 : 3). The mixed solution was refluxed for 5 hours under an argon atmosphere while adding 50 µl (2.1 µmol) portions of toluene solution containing 43 mmol/I azobisisobutyronitrile every 30 minutes.

After the completion of the reaction, a solvent was removed under reduced pressure, thereby obtaining a residue.

The obtained residue was subjected by means of silica-gel column chromatography (an eluant; n-hexane : ethyl acetate 75 : 25 to 33 : 67), thereby removing polymer and tin compounds. Fractions containing a desired product were collected and purified by means of a preparative silica gel chromatography (an eluant is a mixed solution n-hexane and ethyl acetate in a ratio of 50 : 50). A desired compound was obtained in a yield of 10 mg (72 %).

Physical properties of the obtained product are as follows:
¹H-NMR (CDCl₃): δ (ppm)
8.83 (1 H, br, NH)
7.84 (1 H, d, J = 8.1 Hz, H - 6)
6.18 (1 H, dd, J = 7.7, 1.8 Hz, H - 1')
5.67 (1 H, dd, J = 8.1, 2.2 Hz, H - 5)
4.00 - 3.81 (3H, m, H - 4', H - 5')
2.54 (1 H, ddd, J = 14.6, 7.7, 4.7 Hz, H - 2')
2.01 (1 H, d, J = 14.6 Hz, H - 2')
1.05 - 0.85 (18H, m, methyl)
0.75 - 0.51 (12H, m, methylene)

## Claims

1. A process for producing a compound represented by a general formula (1), 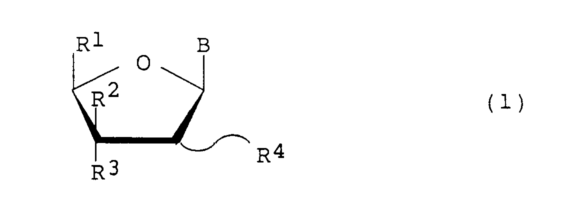 wherein R¹ is a hydrogen atom, an alkyl group, or R⁶0CH₂-, where R⁶ is a protecting group of a hydroxyl group;
R² is a hydrogen atom or a substituent made up of combinations of elements belonging to from the 14th group (the IV group) to the 17th group (the VII group) of the periodic table;
R³ is a hydrogen atom or a substituent made up of combinations of elements belonging to from the 14th group (the IV group) to the 17th group (the VII group) of the periodic table;
R⁴ is a hydrogen atom or a substituent made up of combinations of elements belonging to from the 14th group (the IV group) to the 17th group (the VII group) of the periodic table; and
B is a nucleic acid base;
which comprises
(a) producing a substituted 2-halogenotetrahydrofuran derivative represented by a general formula (4) by reacting a 2,3-dihydrofuran derivative represented by a general formula (2) with an organic halogenated compound represented by a general formula (3) containing an element (A) belonging to the 16th group (the VI group) as shown below, 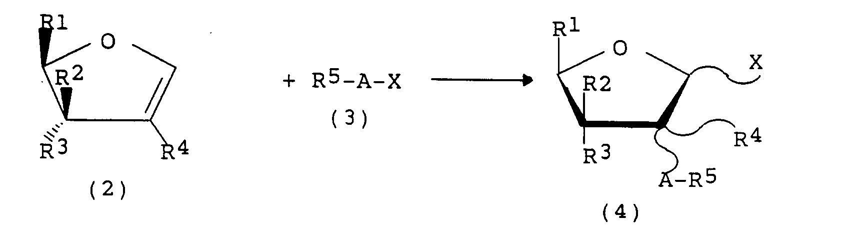
Wherein, the element (A) is an element belonging to the 16th group (the VI group) in the periodic table; X is a halogen atom; and R¹, R², R³, and R⁴ are defined hereinabove; and
R⁵ is an alkyl group or a phenyl group a part of which may be replaced with a substituent, (b) producing a substituted nucleoside derivative represented by a general formula (5) by condensing a compound (4) with a nucleic acid base (B) as shown below, 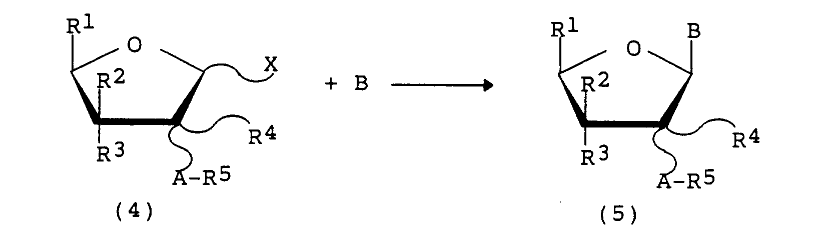
Wherein R¹, R², R³, R⁴, R⁵ A, B, and X are defined hereinabove; and (c) producing a nucleoside derivative represented by a general formula (1) by reducing a chemical compound (5), 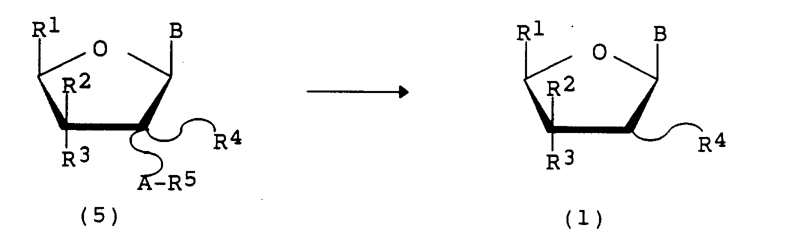
Wherein R¹, R², R³, R⁴, R⁵, A, and B are defined hereinabove.
